# EUROPÄISCHE PATENTANMELDUNG

(11) **EP 0 761 224 A1**
(43) Veröffentlichungstag der Anmeldung: **12.03.1997**
(21) Anmeldenummer: 96112850.1
(22) Anmeldetag: 09.08.1996
(51) Int. Cl.: A61K 31/505

(54) **Verwendung von Orotsäure zur Herstellung eines Arzneimittels zur Prävention und/oder Therapie des Remodelling bei Patienten mit akutem Infarkt**

(30) Priorität: 17.08.1995 DE 19530278
(71) Anmelder: WÖRWAG PHARMA GmbH, 71034 Böblingen (DE)
(72) Erfinder: Wörwag, Fritz, Dr., 70192 Stuttgart (DE)
(74) Vertreter: Weller, Wolfgang, Dr.rer.nat.

(57) **Zusammenfassung**

Verwendung von Orotsäure zur Prävention und/oder Therapie des Remodelling bei Patienten mit akutem Infarkt. Die Orotsäure kann vorteilhafterweise zur Therapie und/oder Prävention der Dilatation des linken Ventrikels des Herzens verwendet werden.

## Beschreibung

Die vorliegende Erfindung betrifft die Verwendung eines Produktes zur Prävention und/oder Therapie des Remodelling bei Patienten mit akutem Infarkt.

Unter einem Infarkt wird eine Nekrose eines umschriebenen Gewebebezirkes, z. B. der Herzmuskulatur verstanden, die meist als akute Komplikation bei chronischer Minderdurchblutung, z. B. bedingt durch eine Koronare Herzkrankheit auftritt.

Am häufigsten wird ein Infarkt in dem meistbeanspruchten Muskel des menschlichen Körpers, dem Herzmuskel beobachtet. Es können jedoch auch andere Regionen des Körpers betroffen sein, wie z. B. Nieren oder Hirn.

Als Ursachen für den Myokardinfarkt kommen eine anhaltende kritische Mangeldurchblutung bei Koronarinsuffizienz oder länger andauernde Koronargefäßspasmen in Frage. Der Myokardinfarkt manifestiert sich häufig bei körperlicher oder psychischer Belastung infolge einer Steigerung des Sauerstoffbedarfes des Herzmuskels oder bei akuter Unterbrechung der Blutversorgung vor allem bei Koronarsklerose mit thrombotischem Verschluß eines Koronargefäßes.

Als Remodelling werden ungünstige und unerwünschte strukturelle Umbauprozesse der Herzmuskulatur und des zwischengelagerten Bindegewebes nach einem Herzinfarkt verstanden. Diese Umbauprozesse beginnen zunächst, ohne Symptome zu erzeugen. Letztendlich können diese Vorgänge jedoch unerkannt und unbehandelt zu einer symptomatischen Herzinsuffizienz oder zum sofortigen Herztod führen.

Bei vielen Infarktpatienten zeigt sich nach einem Myokardinfarkt ein Remodelling des Herzens in einer unerwünschten Größenzunahme des linken Ventrikels des Herzens. Dabei werden abgestorbene kontraktile Elemente, die für die Pumpfunktion des Herzens entscheidend sind, durch Bindegewebe ersetzt. Dadurch nimmt das Herz zwar an Größe zu, trotz dieser Dilatation verliert es jedoch an Pumpkraft.

Weitere Faktoren, die das Remodelling begünstigen, also die Dilatation des Herzens fördern und damit den Postinfarktverlauf verschlechtern, sind z. B. eine gestörte Glukoseutilisation, ein gestörter Fettstoffwechsel und/oder eine gestörte Elektrolytbilanz, die Herzrhythmusstörungen begünstigt.

Um den klinischen Krankheitsverlauf nach Myokardinfarkt zu verbessern, soll bei Patienten mit einem Myokardinfarkt der Prozeß des Remodelling des Herzens verhindert oder verzögert werden.

Bisher erfolgte die Behandlung durch rasche Wiederöffnung der verschlossenen Infarktgefäße und durch ACE-Hemmer, womit die bekannten unerwünschten Risiken und/oder Nebenwirkungen einhergehen.

Vor diesem Hintergrund ist es Aufgabe der vorliegenden Erfindung, ein Produkt zur Behandlung/Verbesserung des Remodelling bei Patienten mit akutem Infarkt bereitzustellen.

Erfindungsgemäß wird diese Aufgabe dadurch gelöst, daß Orotsäure zur Prävention und/oder Therapie des Remodelling bei Patienten mit akutem Infarkt verwendet wird. Dabei ist es bevorzugt, wenn Orotsäure zur Therapie und/oder Prävention der Dilatation des linken Ventrikels des Herzens verwendet wird.

Orotsäure wird bisher in Salzform als sogenannter Mineral- oder Elektrolyt-Schlepper eingesetzt und dient dazu, bestimmte Kationen in die Zelle zu transportieren. So ist es aus der DE-OS-24 10 181 beispielsweise bekannt, Lithiumorotat zu verwenden, um Natrium, das bei der Regulierung des Wasserhaushaltes eine Rolle spielt, in der Zelle durch Lithium zu ersetzen. Lithium wird hier in Form des Salzes der Orotsäure verwendet, da dieses den Transport des Lithiums in die Zelle bewirken kann.

Durch die Deplazierung des Natriums durch Lithium wird die Menge an gespeichertem Wasser reduziert. Dieser Effekt wird zur Therapie von Hypertonie, Arteriosklerose, Gefäß- und Gewebserkrankungen insbesondere im zerebralen Bereich sowie zur Dauer- und Schutzbehandlung von Migräne ausgenutzt.

Aus der Deutschen Apothekerzeitung Nr. 32, 10. August 1995, Seiten 52-53 "Magnesium und Orotsäure gegen Streß und Leistungsschwäche" ist bekannt, daß Orotsäure kardioprotektive, also gefäßschützende Eigenschaften zeigt. Das bedeutet, eine weitere Gefäßverengung, die zu einem erneuten Infarkt führen könnte, kann verhindert werden. Daraus ist aber nicht bekannt, daß ein Remodelling, also die Veränderung der Struktur eines infarktgeschädigten Muskels, verhindert werden kann. Aus "Orotic Acid Cardiol, International Symposium, Orotic Acids Magnesium Orotate (1992), Meeting Date 1991, Seiten 25-35, Thieme Verlag, Stuttgart ist bekannt, daß Orotsäure die Toleranz eines infarktgeschädigten Herzens gegenüber Ischämien verbessert werden kann. Aus J. Mol. Cell. Cardiol., 1989, 21 (8), Seiten 751-754 ist es bekannt, daß ein infarktgeschädigtes Herz einen künstlich induzierten reversiblen Herzstillstand (Kardioplegie) besser toleriert, wenn zuvor mit Orotsäure behandelt wurde. Auch diese Druckschrift geht in die Richtung, daß Orotsäure kardioprotektive Eigenschaften hat, Hinweise darauf, daß Orotsäure Auswirkungen auf das Remodelling hat, sind nicht enthalten oder nahegelegt.

In überraschender Weise hat sich gezeigt, daß Orotsäure die Auswirkungen des Remodelling verhindert und auch noch zu einer Verbesserung der Stoffwechselaktivität nicht nur im infarzierten Gewebe führt, sondern darüber hinaus in dem Areal, welches das pathologisch veränderte Gewebe umgibt. Dadurch werden auch die Folgen dieser Umbauprozesse verhindert und das Auftreten von Herzinsuffizienz reduziert.

Die Orotsäure verhindert bzw. verringert dabei insbesondere die Dilatation des linken Ventrikels des Herzens. Dies ist insbesondere deswegen wichtig, da das Remodelling am Herzen, wie eingangs erwähnt, durch die Umstrukturierung der Herzmuskulatur zu einer Vergrößerung des linken Ventrikels führt, da beim Remodelling der Muskel durch Bindegewebe ersetzt wird. Eine Vergrößerung des linken Ventrikels führt zu einer Schwächung des Herzens.

Damit gewinnt Orotsäure in der Sekundärprävention des Herzinfarktes einen hohen therapeutischen Stellenwert.

Die Orotsäure kann dabei direkt oder in Form eines ihrer Salze verwendet werden, wobei vorzugsweise Magnesiumorotat eingesetzt wird.

In einer Studie an Patienten mit Koronarer Herzkrankheit und größtenteils nach einem Myokardinfarkt konnte echokardiographisch gezeigt werden, daß durch Medikation mit Magnesiumorotat auch eine bereits eingetretene Umstrukturierung des Herzens verbessert werden konnte.

Weiter zeigte sich bei dieser Studie unter Magnesiumorotat ein signifikant geringerer Anstieg des atrialen natriuretischen Faktors (ANF) unter Belastung. Dies ist ein weiterer Laborchemischer Beleg dafür, daß sich durch Magnesiumorotat der Prozeß des Remodelling gebessert hat, denn es zeigt sich, daß unter Magnesiumorotat-Gabe bei Belastung ein verminderter Füllungsdruck auftritt, was ein Zeichen einer verbesserten Myokardkontraktilität ist.

Magnesiumorotat wirkt auch als Membranschutzmittel, was für Infarktpatienten besonders wichtig ist, da durch den Myokardinfarkt Ischämien auftreten, die zum Auslecken der Zellen führen und Elektrolytverluste bedingen, die zu Arrhythmien führen.

Da Magnesiumorotat die Elektrolytbilanz verbessert, wird den Herzrhythmusstörungen entgegengewirkt.

Ferner konnte in Tierexperimenten gezeigt werden, daß Orotsäure arteriosklerotischen Gefäßveränderungen unter experimentellem Cholesterinstreß entgegenwirkt. Dabei zeigte sich, daß die Wirkung der reinen Orotsäure deutlich besser war als die von MgCl₂, so daß der Wirkeffekt bei Magnesiumorotat nicht primär auf das Magnesium, sondern auf die Orotsäure zurückzuführen ist.

Aus all diesen Studien ergibt sich, daß Orotsäure selbst eine positive Wirkung auf die Faktoren hat, die das Remodelling begünstigen, so daß Orotsäure durch eine Verringerung dieser Faktoren die Dilatation des Herzens verhindert und somit für einen günstigen Postinfarktverlauf sorgt.

Dies ist als überraschend anzusehen und war in dieser Deutlichkeit und Ausprägung nicht zu erwarten.

In einem Ausführungsbeispiel ist es dann bevorzugt, wenn eine Mischung aus Orotsäure und einer Magnesiumverbindung verwendet wird.

Hier ist von Vorteil, daß ein synergistischer Effekt eintritt, zum einen wird die Wirkung der Orotsäure ausgenutzt, wobei zusätzlich eine Magnesiumverbindung eingesetzt wird, um auch deren Wirkung mit zu verwenden.

Die Mischung besteht dabei vorzugsweise aus einem Salz der Orotsäure und einer Magnesiumverbindung, wobei auch eine Mischung aus Orotsäure und Magnesiumorotat verwendet werden kann.

Auch hier ist von Vorteil, daß neben der reinen Orotsäure Magnesium in dem herzustellenden Arzneimittel/Präparat/Produkt zu finden ist.

Das aus Orotsäure und/oder ihren Salzen hergestellte Arzneimittel/Produkt kann dabei in allen Darreichungsformen auftreten.

Die Erfindung wird nachfolgend anhand einiger Studien und im Zusammenhang mit den Figuren näher beschrieben und erläutert.

Es zeigen:
- Fig. 1: den Vergleich des ANF-Anstieges unter Belastung bei einer Magnesiumorotat-Gruppe und einer Kontrollgruppe;
und
- Fig. 2: Indizes arteriosklerotischer Gefäßveränderungen unter experimentellem "Cholesterinstreß" bei Kaninchen.

### Beispiel 1: Untersuchung der Wirkung von Magnesiumorotat auf die Belastbarkeit von Patienten mit Koronarer Herzkrankheit

Im Rahmen einer doppelblinden, placebokontrollierten Studie wurden 14 Patienten einer ambulanten Herz-Sportgruppe einer vierwöchigen Therapiephase unterworfen. Alle 14 Patienten wiesen echokardiographisch ein linksventrikuläres enddiastolisches Volumen (LVEDV) von mindestens 100 ml als Zeichen einer eingeschränkten linksventrikulären Funktion auf und hatten z.T. einen Myokardinfarkt erlitten.

Nach einer fahrradergometrischen Ausgangsuntersuchung wurden unter Fortsetzung der vor Studienbeginn bestandenen Medikation zusätzlich 3 x 2 Tabletten à 500 mg Magnesiumorotat bzw. in der Kontrollgruppe Placebo appliziert. Im Anschluß an diese Applikationsphase erfolgt eine Abschlußuntersuchung.

In der Verumgruppe verringerte sich das linksventrikuläre endsystolische Volumen (LVEDV) um 25,3 ml (p = 0, 016), die Ejektionsfraktion zeigte eine Zunahme von 42,6 % auf 51,7 % (p = 0,034). Ferner zeigte sich eine schwach signifikante Abnahme des LVEDV von 130,7 ml auf 103 ml (p = 0,053).

Anhand der beschrieben verbesserten Leistung des Herzens konnte auf eine Verbesserung des Remodelling des Herzens geschlossen werden.

Ferner wurde noch der atriale natriuretische Faktor (ANF) bestimmt, der bei der Regelung des Blutvolumens und Salzhaushaltes mitwirkt. Der Vergleich des ANF-Anstiegs unter Belastung zwischen Verum- und Kontrollgruppe zeigte in der Abschlußuntersuchung signifikante Unterschiede (p = 0,049).

Dies ist ein weiterer laborchemischer Beleg dafür, daß sich durch Magnesiumorotat der Prozeß des Remodelling gebessert hat, denn es zeigt sich, daß unter Magnesiumorotat-Gabe bei Belastung ein verminderter Füllungsdruck auftritt, was ein Zeichen einer verbesserten Myokardkontraktilität ist.

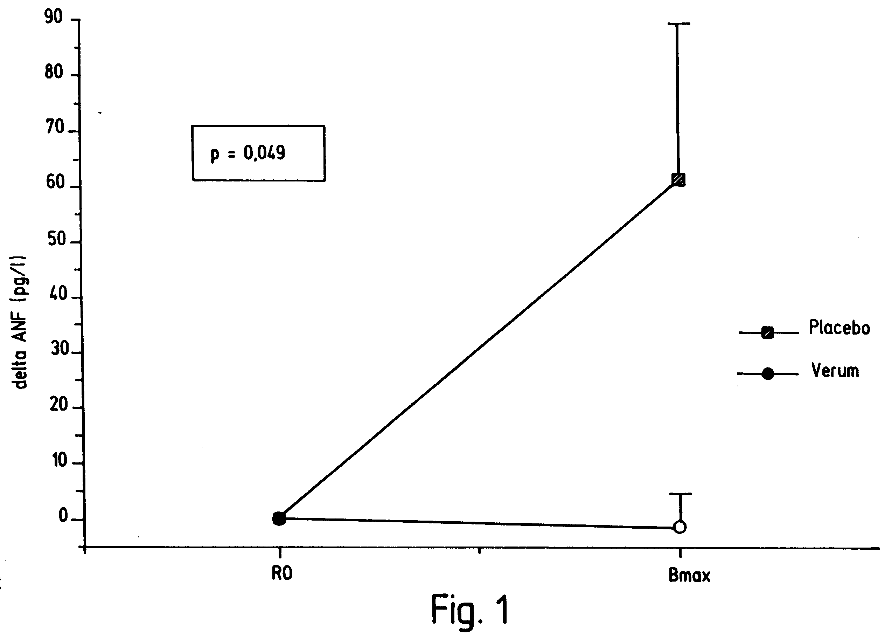

In Fig. 1 ist die Differenz des ANF-Anstieges unter Belastung (delta ANF) wiedergegeben.

### Beispiel 2: Untersuchung der Wirkung von Orotsäure und Magnesiumorotat in einem Arteriosklerose-Modell

In einer Versuchsreihe mit einem Arteriosklerose-Modell wurden Kaninchen über 112 Tage einer zweiprozentigen Cholesterindiät ausgesetzt und mit äquimolaren Mengen von Magnesiumchlorid, Orotsäure und Magnesiumorotat bzw. in der Kontrollgruppe überhaupt nicht weiter behandelt. Zusätzlich zu histologischen Untersuchungen der Koronaarterien sowie der Aorta femoralis und renalis wurde der Zustand der Aorta an jeweils mehreren Stellen auch planimetrisch-photographisch dokumentiert. Mittels einer computergestützten, digitalen Methode der Oberflächenmessung ("density slice"-Methode) erhält man hierbei dreidimensionale Bilder, mit deren Hilfe auch eine Quantifizierung der cholesterininduzierten Lumeneinengungen möglich ist.

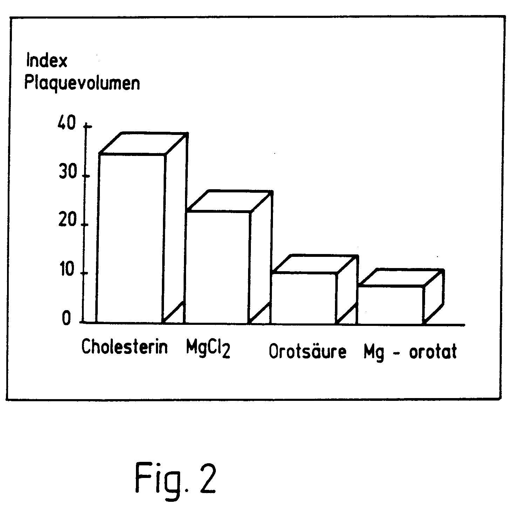

In Fig. 2 ist das Ergebnis dieser Untersuchung dargestellt. Es ist zu erkennen, daß die Gefäßverengungen in der unbehandelten Kontrollgruppe mit einem Index von 34, 36 am ausgeprägtesten waren. Die Orotsäure wirkt mit einem Index von 9, 77 deutlich besser den arteriosklerotischen Läsionen entgegen als Magnesiumchlorid (Index 22, 63).

Als bester Schutz erwies sich jedoch Magnesiumorotat mit einem Index von 7, 28.

Diese quantifizierten Ergebnisse wurden durch die licht- bzw. elektronenmikroskopischen Befunde sowie durch enzymhistochemische Untersuchungen bestätigt. Demnach verhütet Magnesiumorotat auch in den Koronarien die sklerotischen Läsionen am besten. Die lumeneinengende, durch die Cholesterinfütterung induzierte Proliferation von Schaumzellen war unter dieser Medikation weitaus am geringsten ausgeprägt.

Aus dieser Untersuchung ergibt sich, daß Orotsäure selbst arteriosklerotische Gefäßveränderungen entscheidend verhütet und daß diese Wirkung durch Zugabe von Magnesium weiter gesteigert werden kann.

Beispiel 1 zeigt, daß durch Magnesiumorotat bei Patienten mit Koronarer Herzkrankheit und dabei häufig einem vorausgegangenen Myokardinfarkt die Leistung des Herzens und die Myokardkontraktilität direkt verbessert werden konnten. Damit werden die negativen Folgen eines schädlichen Remodellings reduziert bzw. aufgehoben.

Die weiteren Unterscuhungen zeigen, daß Orotsäure selbst und besser noch Magnesiumorotat sich positiv auf eine gestörte Glukoseutilation, einen gestörten Fettstoffwechsel sowie eine gestörte Elektrolytbilanz auswirken. Damit werden aber die Einflüsse der wichtigsten Faktoren, die das Remodelling begünstigen, verringert oder beseitigt, so daß der Postinfarktverlauf verbessert wird.

Es wird also nicht nur das Remodelling selbst, wenn es schon eingesetzt hat, reduziert, sondern auch gleichzeitig das Remodelling begünstigende Faktoren verringert.

## Patentansprüche

1. Verwendung von Orotsäure zur Herstellung eines Arzneimittels zur Prävention und/oder Therapie des Remodelling bei Patienten mit akutem Infarkt.

2. Verwendung nach Anspruch 1, dadurch gekennzeichnet, daß Orotsäure zur Therapie und/oder Prävention der Dilatation des linken Ventrikels des Herzens verwendet wird.

3. Verwendung nach Anspruch 1 oder Anspruch 2, dadurch gekennzeichnet, daß ein Salz der Orotsäure verwendet wird.

4. Verwendung nach Anspruch 3, dadurch gekennzeichnet, daß Magnesiumorotat verwendet wird.

5. Verwendung nach einem der Ansprüche 1 bis 4, dadurch gekennzeichnet, daß eine Mischung aus Orotsäure und einer Magnesiumverbindung verwendet wird.

6. Verwendung nach einem der Ansprüche 1 bis 5, dadurch gekennzeichnet, daß eine Mischung aus einem Salz der Orotsäure und einer Magnesiumverbindung verwendet wird.

7. Verwendung nach Anspruch 5 oder Anspruch 6, dadurch gekennzeichnet, daß eine Mischung aus Orotsäure und Magnesiumorotat verwendet wird.
